# DEMANDE DE BREVET EUROPEEN

(11) **EP 1 068 857 A1**
(43) Date de publication de la demande: **17.01.2001**
(21) Numéro de dépôt: 00401550.9
(22) Date de dépôt: 31.05.2000
(51) Int. Cl.: A61K 7/06, A61K 7/02, A61K 7/043

(54) **Utilisation en cosmétique de copolymères amphotères réticulés ou branchés; compositions mises en oeuvre**

(30) Priorité: 16.07.1999 FR 9909266
(71) Demandeur: L'OREAL, 75008 Paris (FR)
(72) Inventeur: Sebag, Henri, 75016 Paris (FR); Dubief, Claude, 78150 Le Chesnay (FR); Restle, Serge, 95390 Saint-Prix (FR)
(74) Mandataire: Dodin, Catherine

(57) **Abrégé**

La présente invention a pour objet l'utilisation dans et pour la préparation de compositions cosmétiques ou dermatologiques, d'au moins un copolymère amphotère réticulé ou branché comprenant au moins un monomère (meth)acrylate ou (meth)acrylamide comportant au moins une chaîne grasse en C₈-C₃₀.

Application au traitement des matières kératiniques et en particulier de la peau, des cheveux, des cils et des ongles.

## Description

La présente invention a trait à l'utilisation de copolymères amphotères réticulés ou branchés comprenant au moins un monomère (meth)acrylate ou (meth)acrylamide comportant au moins une chaîne grasse en C8-C30 dans et pour la préparation de compositions cosmétiques ou dermatologiques ainsi que les compositions mises en oeuvre.

On a déja préconisé dans des compositions cosmétiques pour la peau ou pour le lavage ou le soin des cheveux l'utilisation de polymères conditionneurs, notamment cationiques ou amphotères, pour faciliter le démêlage des cheveux et pour leur communiquer douceur et souplesse. Cependant, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, un raidissement des cheveux, et une adhésion interfibre affectant le coiffage. Ces inconvénients sont accentués dans le cas de cheveux fins, qui manquent de tenue, de nervosité et de volume.

On a également préconisé pour améliorer les propriétés conditionnantes des produits capillaires, l'utilisation de polymères amphotères tels que ceux décrits dans la demande de brevet EP-A-269 243. Cependant, les compositions contenant uniquement ces polymères ne permettent pas d'obtenir une douceur et un démêlage suffisants.

L'un des objectifs de la présente invention est donc d'utiliser dans des compositions cosmétiques ou dermatologiques des polymères apportant de bonnes propriétés cosmétiques telles que le démêlage et la douceur au toucher.

La Demanderesse a découvert de manière surprenante que ces objectifs pouvaient être atteints en utilisant dans et pour la préparation de compositions cosmétiques ou dermatologiques, des copolymères réticulés particuliers.

On a découvert en particulier que les compositions obtenues selon l'invention apportent une amélioration du démêlage (notamment sur cheveux humides) ainsi qu'une amélioration de la douceur des cheveux. En outre, les cheveux ne sont pas alourdis après des applications répétées.

Par ailleurs, les compositions de l'invention appliquées sur la peau apportent une amélioration de la douceur de la peau.

L'invention a pour objet l'utilisation comme produits cosmétiques de polymères amphotères réticulés ou branchés que l'on définiera plus en détail dans la suite de la description.

En effet, les copolymères réticulés ou branchés particuliers de l'invention peuvent notamment être utilisés dans et pour la préparation de produits capillaires pour le conditionnement des cheveux. Ils peuvent également être utilisés dans et pour la préparation de produits hydratants pour le soin ou le maquillage de la peau ou des lèvres pouvant contenir des actifs sans conduire à un dépôt collant.

La présente invention a pour objet l'utilisation dans et pour la préparation de compositions cosmétiques ou dermatologiques, d'au moins un copolymère réticulé ou branché susceptible d'être obtenu par copolymérisation d'un mélange de composés comprenant :
1) au moins un composé de formule (la) ou (Ib): 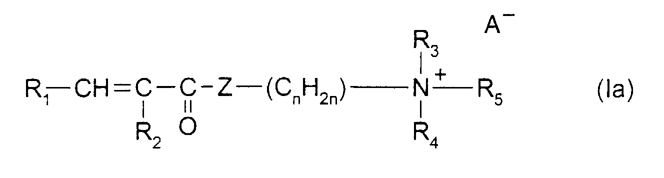 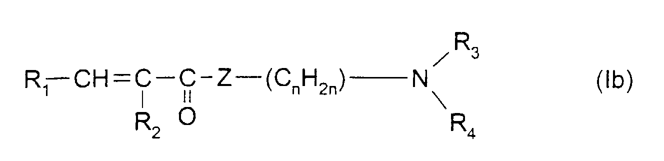
   dans lesquelles R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A⁻ est un anion issu d'un acide organique ou minéral, tel qu'un anion méthosulfate ou un halogénure tel que chlorure ou bromure ;
2) au moins un composé de formule (II): 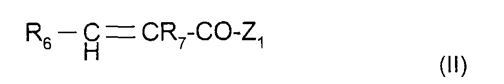 dans laquelle R₆ et R₇, identiques ou différents, représentent un atome d'hydrogène ou un radical méthyle; Z₁ représente un groupe OH ou un groupe NHC(CH₃)₂CH₂SO₃H ;
3) au moins un composé de formule (III): 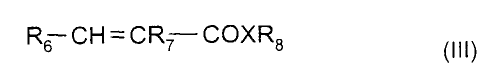 dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
4) au moins un agent de réticulation ou de branchement ;
l'un au moins des monomères de formule (Ia), (Ib) ou (III) comportant au moins une chaîne grasse ayant de 8 à 30 atomes de carbone et lesdits composés (la), (Ib), (II) et (III) pouvant être quaternisés par exemple par un halogénure d'alkyle en C₁-C₄ ou un sulfate de dialkyle en C₁-C₄.

Les composés de formule (la) et (Ib) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide ;
ces composés pouvant être quaternisés par exemple par un halogénure d'alkyle en C₁-C₄ ou un sulfate de dialkyle en C₁-C₄.

Plus particulièrement, le composé de formule (la) est choisi parmi le chlorure d'acrylamidopropyl triméthyl ammonium et le chlorure de méthacrylamidopropyl triméthyl ammonium. Plus avantageusement encore le composé de formule (la) est le chlorure d'acrylamidopropyl-triméthyl ammonium.

Les composés de formule (II) de la présente invention sont choisis, de préférence, dans le groupe constitué par l'acide acrylique, l'acide méthacrylique, l'acide crotonique, l'acide méthyl-2 crotonique, l'acide 2-acrylamido-2-méthylpropane sulfonique et l'acide 2-méthacrylamido-2-méthylpropane sulfonique. Plus particulièrement, le monomère de formule (II) est l'acide acrylique.

Les monomères de formule (III) de la présente invention sont choisis, de préférence, dans le groupe constitué des acrylates ou méthacrylate d'alkyle en C₁₂-C₂₂ et plus particulièrement en C₁₆-C₁₈.

L'agent de réticulation ou de branchement est de préférence choisi parmi le N,N'-méthylène bis-acrylamide, le chlorure de triallyl méthyl ammonium, le méthacrylate d'allyle, le n-méthylolacrylamide, les diméthacrylate de polyéthylène glycols, le diméthacrylate d'éthylène glycol, le diméthacrylate de diéthylène glycol, le diméthacrylate de 1,6-hexanediol et l'allyl sucrose.

Les composés constituant les polymères amphotères de l'invention sont de préférence déjà neutralisés et/ou quaternisés.

La présente invention concerne également des compositions cosmétiques ou dermatologiques contenant dans un milieu cosmétiquement acceptable au moins un copolymère réticulé ou branché susceptible d'être obtenu par copolymérisation d'un mélange de composés décrits ci-dessus.

D'autres objets apparaîtront à la lumière de la description et des exemples qui suivent.

Les copolymères de l'invention sont de préférence des copolymères réticulés ou branchés solubles ou dispersibles dans l'eau, les alcools inférieurs (en C₁-C₄) ou les mélanges d'eau et d'alcool(s) inférieur(s).

Les copolymères réticulés ou branchés conformes à la présente invention, présentent une masse moléculaire moyenne en poids supérieure à 1000, de préférence comprise entre 10000 et 10000000 et encore plus préférentiellement entre 100000 et 8000000.

La viscosité réduite peut être utilisée comme un moyen de détermination de la masse moléculaire en poids selon la présente invention. Les valeurs indiquées ici se déterminent à partir d'un viscosimètre capillaire Ubbelohde avec une concentration en polymère de 0,05% dans une solution 1M de NaCI à pH7 et à 30°C.
On peut aussi utiliser comme méthode alternative la chromatographie par perméation de gel.

De préférence les copolymères de l'invention contiennent de 1 à 99 moles % , plus préférentiellement de 20 à 95 moles% et encore plus préférentiellement de 25 à 75 moles % de composé(s) de formule (la) ou (Ib).

Ils contiennent aussi de préférence de 1 à 80 moles %, plus préférentiellement de 5 à 80 moles% et encore plus préférentiellement de 25 à 75 moles% de composé(s) de formule (II).

La teneur en composé (s) de formule (III) est de préférence comprise entre 0,1 et 70 moles%, plus préférentiellement entre 1 à 50 moles% et encore plus préférentiellement entre 1 à 10 moles%.

L'agent de réticulation ou de branchement est de préférence compris entre 0,0001 et 1 mole% et plus préférentiellement encore entre 0,0001 et 0,1 mole%.

De préférence le rapport molaire entre le ou les composés de formules (la) ou (Ib) et le ou les composés de formule (II) varie de 20 :80 à 95 :5 et plus préférentiellement de 25 :75 à 75 :25.

Les copolymères de l'invention peuvent être préparés par les techniques conventionnelles de polymérisation en solution :
- chargement des composés dans un réacteur muni de moyen d'agitation
- ajustement de la concentration totale en monomères au environs de 10 à 25%
- ajustement du pH de mélange entre 3 et 6,5
- purge à l'azote
- initialisation de la polymérisation avec du persulfate de sodium
- dilution à l'eau en présence de bisulfite de sodium après atteinte du pic exothermique, ceci pour atteindre une concentration de l'ordre de 4 à 8% en polymère.

Les compositions cosmétiques et dermatologiques selon l'invention contiennent donc dans un support cosmétiquement acceptable les polymères tels que décrits ci-dessous, pour des applications aussi variées que celles rencontrées par exemple dans le domaine du capillaire, du maquillage ou bien encore des soins de la peau, ou de tout autre domaine cosmétique dans lequel l'utilisation d'une substance filmogène est désirable ou recherchée.

Les copolymères réticulés ou branchés selon l'invention peuvent être utilisés seuls comme polymères conditionneurs ou bien comme additif à des agents conditionneurs conventionnels dans et pour la préparation de compositions cosmétiques ou dermatologiques.

La concentration en copolymère dans les compositions cosmétiques ou dermatologiques de l'invention est généralement comprise entre 0,01 et 50%, et de préférence entre 0,1 et 20% en poids total de la composition. Elle varie selon l'application cosmétique ou dermatologique envisagée.

Les compositions selon l'invention peuvent se présenter sous toutes les formes appropriées pour une application topique, notamment sous forme de solutions du type lotion ou sérum ; sous forme de gels ; sous forme d'émulsions obtenues par dispersion d'une phase grasse dans une phase aqueuse (H/E) ou inversement (E/H), de consistance liquide plus ou moins épaissie telles que des laits, des crèmes plus ou moins onctueuses, sous forme de dispersions. Ces compositions sont préparées selon les méthodes usuelles.

Les compositions selon l'invention peuvent être utilisées comme produits capillaires notamment pour le lavage, le soin, le conditionnement, le maintien de la coiffure , la mise en forme , la coloration , la décoloration , la déformation permanente ou le défrisage des cheveux.

Les compositions non-rincées capillaires selon l'invention sont de préférence des produits de coiffage tels que des lotions de mise en plis, des lotions pour le brushing, des compositions de fixation et de coiffage telles que des laques ou spray. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une mousse pour la fixation ou le traitement des cheveux.

Les compositions capillaires rincées sont plus particulièrement des shampooings coiffants et/ou conditionneurs ou bien des après-shampooings à rincer.

Les compositions de l'invention peuvent être également utilisées comme produit de soin et/ou l'hygiène tels que des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions de l'invention peuvent être également utilisées comme démaquillants. Les compositions peuvent être des produits pour le maquillage et plus particulièrement des vernis à ongles ou des bases de soin pour les ongles.

Le support cosmétiquement acceptable des compositions selon l'invention est de préférence constitué d'eau, d'un ou plusieurs solvants organiques cosmétiquement acceptables ou bien d'un mélange d'eau et d'un ou plusieurs solvants organiques cosmétiquement acceptables.

Parmi ces solvants organiques, on utilise plus particulièrement les alcools inférieurs en C₁-C₄ tels que l'éthanol.

Les copolymères réticulés ou branchés selon l'invention sont dissous ou en dispersion dans le support des compositions de l'invention.

Les compositions peuvent en outre, et bien entendu, contenir divers adjuvants destinés à la rendre acceptable dans une application cosmétique particulière .

Les compositions selon l'invention peuvent contenir des additifs cosmétiques conventionnels choisis parmi les corps gras tels que les huiles minérales, animales, les cires animales, fossiles, minérales ou de synthèse, des solvants organiques, des agents épaississants, des adoucissants, des agents anti-mousse, des agents hydratants, des humectants, des agents traitants (agents anti-chute, ...), des antiperspirants, des agents alcanisants, des filtres solaires UV-A ou UV-B ou à bande large, des colorants, des pigments,des agents oxydants, des agents réducteurs, des parfums, des plastifiants, des conservateurs, des polymères organiques anioniques,cationiques, non-ioniques ou amphotères et des agents propulseurs lorsque les compositions se présentent sous forme aérosol.

De manière avantageuse on associera les polymères de l'invention aux composés suivants, seuls ou en mélanges :
- esters d'acides carboxyliques insolubles dans l'eau
- agents antipelliculaires
- silicones et en particulier silicones insolubles dans l'eau
- polymères fixants ou conditionneurs
- agents conditionneurs choisis parmi les huiles de synthèse, les huiles végétales, les cires végétales et les composés de type céramide.

Bien entendu, l'homme de l'art veillera à choisir le ou les éventuels composés complémentaires mentionnés ci-avant, de manière telle que les propriétés avantageuses attachées intrinsèquement aux compositions selon l'invention ne soient pas, ou substantiellement pas, altérées par la ou les adjonctions envisagées.

L'invention a également pour objet un procédé de traitement cosmétique des matières kératiniques telles que la peau, les cheveux, le cuir chevelu, les cils, les sourcils, les ongles, les lèvres, caractérisé en ce qu'il consiste à appliquer sur ces dernières une composition telle que définie ci-dessus.

### EXEMPLES DE FORMULATION

### Exemple 1 comparatif :

| | **A** **Hors invention** | **B** **Selon l'invention** |
|---|---|---|
| Lauryl éther sulfate à 2,2 moles d'oxyde d'éthylène | 8% MA | 8% MA |
| N-cocoyl amidoéthyl N ethoxycarboxyméthyl glycinate de sodium | 4% MA | 4% MA |

| Polymère 1: | | |
|---|---|---|
| MAPTAC(49)/AA(49)/SMA(2) | 1% MA | - |

| Polymère 2 : | | |
|---|---|---|
| MAPTAC(49)/AA(49)/SMA(1,9994)/MBA(0,0006) | - | 1% MA |
| Eau qsp | 100 | 100 |
| MAPTAC= Chlorure de méthacrylamidopropyltriméthylammonium | | |
| AA = Acide acrylique | | |
| SMA = Méthacrylate de stéaryle | | |
| MBA = N,N' méthylènebisacrylamide | | |

Les compositions A et B sont appliquées à raison de 1 g sur des mèches de cheveux naturels de 2,7g. Après 5 minutes de pose, les cheveux sont rincés et séchés.

### Résultats :

Un test comparatif portant sur l'évaluation du caractère lisse des mèches a été réalisé sur mèches à la fois mouillées et sèches avec un panel de 10 testeurs.

Dans une échelle où les différences d'état de lissage sont évaluées de 1 (différence très peu marquée) à 4(différence très marquée) on constate pour l'ensemble des testeurs une somme des différences de +22 en faveur de la composition B selon l'invention.

Cette composition conduit donc à un lissage des cheveux significativement plus important que celui obtenu avec la composition A hors invention.

### Exemple 2 :

| | |
|---|---|
| Lauryl éther sulfate à 2,2 moles d'oxyde d'éthylèhe | 8% MA |
| N-cocoyl amidoéthyl N ethoxycarboxyméthyl | |
| glycinate de sodium | 4% MA |
| Silicone PDMS (500 000) | 1,5% |
| Polymère MAPTAC(49)/AA(49)/SMA(1,9994)/MBA(0,0006) 1% MA | |
| Eau qsp | 100 |

Cette composition appliquée sur cheveux conduit après rinçage à des propriétés cosmétiques de conditionnement de la fibre capillaire remarquables.

## Revendications

1. Utilisation dans et pour la préparation de compositions cosmétiques ou dermatologiques, d'au moins un copolymère réticulé ou branché obtenu par copolymérisation d'un mélange de composés comprenant :
1) au moins un composé de formule (la) ou (Ib): 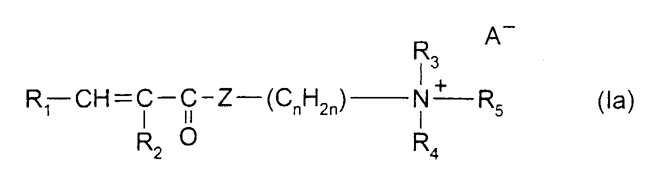 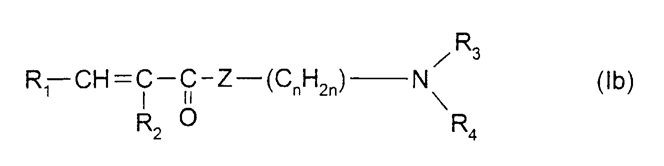
dans lesquelles R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
Z représente un groupe NH ou un atome d'oxygène,
n est un nombre entier de 2 à 5,
A⁻ est un anion issu d'un acide organique ou minéral, tel qu'un anion méthosulfate ou un halogénure tel que chlorure ou bromure ;
2) au moins un composé de formule (II): 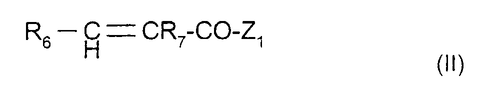
et dans laquelle R₆ et R₇, identiques ou différents, représentent un atome d'hydrogène ou un radical méthyle ;
Z₁ représente un groupe OH ou un groupe NHC(CH₃)₂CH₂SO₃H ;
3) au moins un composé de formule (III): 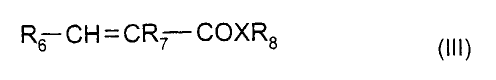 dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
4) au moins un agent de réticulation ou de branchement,
l'un au moins des composés de formule (la), (Ib) ou (III) comportant au moins une chaîne grasse ayant de 8 à 30 atomes de carbone et lesdit composés de formule (la), (Ib), (Il) ou (III) pouvant être quaternisés.

2. Utilisation selon la revendication 1, selon laquelle les composés de formule (la) et (Ib) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
lesdits composés de formule (la) et (Ib) pouvant être quaternisés.

3. Utilisation selon la revendication 1 ou 2, selon laquelle le composé de formule (la) est choisi parmi le chlorure d'acrylamidopropyl triméthyl ammonium et le chlorure de méthacrylamidopropyl triméthyl ammonium.

4. Utilisation selon l'une quelconque des revendications 1 à 3, selon laquelle les composés de formule (II) de la présente invention sont choisis dans le groupe constitué par l'acide acrylique, l'acide méthacrylique, l'acide crotonique, l'acide méthyl-2 crotonique, l'acide 2-acrylamido-2-méthylpropane sulfonique et l'acide 2-méthacrylamido-2-méthylpropane sulfonique.

5. Utilisation selon l'une quelconque des revendications 1 à 4, selon laquelle les composés de formule (III) de la présente invention sont choisis dans le groupe constitué des acrylates ou méthacrylate d'alkyle en C₁₂-C₂₂ et plus particulièrement en C₁₆-C₁₈.

6. Utilisation selon l'une quelconque des revendications 1 à 5, selon laquelle l'agent de réticulation ou de branchement est choisi parmi le N,N'-méthylène bis-acrylamide, le chlorure de triallyl méthyl ammonium, le méthacrylate d'allyle, le n-méthylolacrylamide, les diméthacrylates de polyéthylène glycol, le diméthacrylate d'éthylène glycol, le diméthacrylate de diéthylène glycol, le diméthacrylate de 1,6-hexanediol et l'allyl sucrose.

7. Utilisation selon l'une quelconque des revendications 1 à 6, selon laquelle les copolymères de l'invention contiennent de 1 à 99 moles % , plus préférentiellement de 20 à 95 moles% et encore plus préférentiellement de 25 à 75 moles % de composé(s) de formule (Ia) ou (Ib).

8. Utilisation selon l'une quelconque des revendications 1 à 7, selon laquelle les copolymères de l'invention contiennent de 1 à 80 moles %, plus préférentiellement de 5 à 80 moles% et encore plus préférentiellement de 25 à 75 moles% de composé(s) de formule (II).

9. Utilisation selon l'une quelconque des revendications 1 à 8 selon laquelle les copolymères de l'invention contiennent de 0,1 à 70 moles%, plus préférentiellement de 1 à 50 moles% et encore plus préférentiellement de 1 à 10 moles% de composé (s) de formule (III).

10. Utilisation selon l'une quelconque des revendications 1 à 9 selon laquelle les copolymères de l'invention contiennent de 0,0001 à 1 mole% et plus préférentiellement de 0,0001 à 0,1 mole% d'agent de réticulation ou de branchement.

11. Utilisation selon l'une quelconque des revendications 1 à 10 selon laquelle dans les copolymères de l'invention le rapport molaire entre le ou les composés de formules (la) ou (Ib) et le ou les composés de formule (II) varie de 20 :80 à 95 :5 et plus préférentiellement de 25 :75 à 75 :25.

12. Utilisation selon l'une quelconque des revendications 1 à 11 selon laquelle les copolymères de l'invention présentent une masse moléculaire moyenne en poids ,déterminée par viscométrie, supérieure à 1000, de préférence comprise entre 10000 et 10000000 et encore plus préférentiellement entre 100000 et 8000000.

13. Composition cosmétique ou dermatologique, caractérisée par le fait qu'elle contient dans un support cosmétiquement acceptable au moins un copolymère tel que défini selon l'une quelconque des revendications 1 à 12.

14. Composition selon la revendication 13, caractérisée par le fait que la concentration en copolymère dans les compositions cosmétiques est généralement comprise entre 0,01 et 50%, et de préférence entre 0,1 et 20% par rapport au poids total de la composition.

15. Composition selon la revendication 13 ou 14, caractérisée par le fait que le support cosmétiquement acceptable est constitué d'eau, d'un ou plusieurs solvants organiques cosmétiquement acceptables ou bien d'un mélange d'eau et d'un ou plusieurs solvants organiques cosmétiquement acceptables.

16. Composition selon la revendication 15, caractérisée par le fait que le ou les solvants organiques cosmétiquement acceptables sont choisis parmi les alcools inférieurs en C₁-C₄.

17. Composition selon l'une quelconque des revendications 13 à 16, caractérisée par le fait que le copolymère est dissous ou en dispersion dans le support de la composition.

18. Composition selon l'une quelconque des revendications 13 à 17, caractérisée par le fait qu'elle contient au moins un ester d'acide carboxylique insoluble dans l'eau.

19. Composition selon l'une quelconque des revendications 13 à 18, caractérisée par le fait qu'elle contient au moins un agent antipelliculaire.

20. Composition selon l'une quelconque des revendications 13 à 19, caractérisée par le fait qu'elle contient au moins une silicone.

21. Composition selon la revendications 20, caractérisée par le fait que la silicone est insoluble dans l'eau.

22. Composition selon l'une quelconque des revendications 13 à 21, caractérisée par le fait qu'elle contient au moins un polymère fixant ou conditionneur.

23. Composition selon l'une quelconque des revendications 13 à 22, caractérisée par le fait qu'elle contient au moins un agent conditionneur choisi parmi les huiles de synthèse, les huiles végétales, les cires végétales et les composés de type céramide.

24. Composition selon l'une quelconque des revendications 13 à 23, caractérisée par le fait qu'elle contient des additifs cosmétiques conventionnels choisis parmi les corps gras tels que les huiles minérales, animales, les cires animales, fossiles, minérales ou de synthèse, des solvants organiques, des agents épaississants, des adoucissants, des agents anti-mousse, des agents hydratants, des humectants, des agents traitants, des antiperspirants, des agents alcanisants, des agents acidifiants, des filtres solaires UV-A ou UV-B ou à bande large, des colorants, des pigments, des oxydants, des réducteurs, des parfums, des plastifiants, des conservateurs, des polymères organiques anioniques, non-ioniques ou amphotères et les agents propulseurs.

25. Composition selon l'une quelconque des revendications 13 à 24, caractérisée par le fait qu'elle est utilisée comme produit capillaire pour le lavage, le soin, le conditionnement, le maintien de la coiffure, la mise en forme, la coloration, la décoloration, la déformation permanente ou le défrisage des cheveux.

26. Composition selon la revendication 25, caractérisée par le fait qu'elle est utilisée comme produit de coiffage non-rincé.

27. Composition selon la revendication 25, caractérisée par le fait qu'elle est utilisée comme produit capillaire rincé, en particulier comme shampooing ou comme après-shampooing.

28. Composition selon l'une quelconque des revendications 13 à 24, caractérisée par le fait qu'elle est utilisée comme produit rincé de soin et/ou l'hygiène

29. Composition selon l'une quelconque des revendications 13 à 24, caractérisée par le fait qu'elle est utilisée comme démaquillant.

30. Composition selon l'une quelconque des revendications 13 à 24, caractérisée par le fait qu'elle est utilisée comme produit pour le maquillage.

31. Composition selon la revendication 30, caractérisée par le fait qu'elle est utilisée comme vernis à ongles ou base de soin pour les ongles.

32. Utilisation d'un copolymère tel que défini selon l'une quelconque des revendications 1 à 12 comme agent conditionneur, ou comme additif d'agent conditionneur, dans une et pour la préparation d'une composition cosmétique ou dermatologique.

33. Procédé de traitement des matières kératiniques, caractérisé en ce qu'il consiste à appliquer sur ces dernières une composition telle que définie à l'une quelconque des revendications 13 à 31.
